# EUROPEAN PATENT SPECIFICATION

(11) **EP 1 776 960 B1**
(45) Date of publication and mention of the grant of the patent: **18.03.2009**
(21) Application number: 05425735.7
(22) Date of filing: 20.10.2005
(51) Int. Cl.: A61K 38/39, A61K 31/02, A61K 33/00, A61P 17/02

(54) **Use of collagen in combination with oxygen- and ozone-releasing perfluorocarbons for the preparation of a medicament for the treatment of skin lesions**
Verwendung von Kollagen in Kombination mit Sauerstoff- und Ozon-freisetzenden Perfluorkohlenwasserstoffen zur Herstellung eines Medikaments zur Behandlung von Hautverletzungen
Utilisation de collagène en association avec des perfluorocarbones dégageant de l'oxygène et de l'ozone pour la préparation d'un médicament pour le traitement des lésions de la peau

(43) Date of publication of application: 25.04.2007
(73) Proprietor: Parodi, Filippo Ernestino, 28100 Novara (IT); Papa, Franco, 28040 Lesa NO (IT)
(72) Inventor: Parodi, Filippo Ernestino, 28100 Novara (IT); Papa, Franco, 28040 Lesa NO (IT)
(74) Representative: Lanzoni, Luciano

(56) References cited:
- EP-A- 0 322 249
- WO-A-03/082392
- RIESS, J. G.: "Blood substitutes and other potential biomedical applications of fluorinated colloids" JOURNAL OF FLUORINE CHEMISTRY, vol. 114, no. 2, 28 April 2002 (2002-04-28), pages 119-126, XP004351164

## Description

The present invention relates to a medicament for the treatment of skin lesions. Particularly, the invention relates to a medication and a kit for the topical administration of such a medicament on the damaged skin.

As far as the treatment of skin lesions is concerned, nowadays particularly in hospitals, rest-homes, therapy centers, it occurs:
1) the use of traditional disinfectants and medicament supports, such as iodoform gauzes. The latter are known to have a toxic side effect and to be substantially ineffective in skin lesion care, as explained in the following publications: *"*A case of iodoform poisoning" Lancet. 1903; 1: 960; Tada M., Tada M,, Ishiguro H,, Hirota K., "Consciousness disturbance caused by iodoform absorption in a patient with decubitus ulcer topically treated with iodoform-gauze". No To Shinkei. 2002 Dec;54(12):1051-4); Yamasaki K., Morimoto N., Gion T., Yanaga K., "D. and a subclavian abscess" Lancet., 1997 Nov 1; 350(9087): 1294; Martins CA, Velasco ED, Garcia JA, Caetano R., "Poisoning caused by external use of iodoform" Rev. Hosp, Clin. Fac. Med. Sao Paulo, 1994 Jul-Aug 49 (4):177-8.
2) the use of medications or curative devices for topical administration, which are made of highly expensive materials, particularly in considering the frequency with which such medications are usually replaced. This type of medications is in fact too complex to use and is not cost-effective.

In order that a complete tissue repair occurs, a skin lesion needs specific conditions, such as suitable cellular metabolism values, compatible with the tissue viability, temperature, vascularization, breathing and moisture, as well as the lack of any infection.

In order to achieve such conditions within a conveniently short time, a suitable medicament for topical use should theoretically be able to meet a number of conditions: keeping moist the environment surrounding the lesion, allowing the exudate check, permitting the gas exchange, promoting the thermal insulation, protecting from contaminations, containing no toxic agents, reducing the trauma to tissues upon medication replacement, being comfortable, reducing the frequency of replacements and conforming to irregular surfaces.

It is known the use of non-woven cotton gauzes, which, even though physically protecting the lesion assuring proper transpiration, do not actually help in disinfecting and regenerating the tissues.

It is further known the use of gelatins of glycine, collagen, cellulose polymers and substances having similar properties, which, against a better protection of the lesion from external agent contamination, nevertheless do not allow a proper transpiration/oxygenation of the tissues.

Furthermore, these problems significantly contribute to make worse the conditions of people who in themselves already have serious difficulties in skin regeneration, owing for instance to their old age or specific circulation disorders.

International Patent Application WO 03/082392 discloses a method of treating a microbial infection in a subject, other than by hyberbaric oxygen therapy, which comprises: (a) providing an oxygenating agent; (b) administering an effective dosage of said oxygenating agent to said subject. The oxygenating agent may be selected from oxygen-carrying agents and entrapped oxygen-generating agents. The oxygenating agent may be selected from, inter alia, perfluorocarbons.

European Patent Application EP 0 322 249 A2 discloses a storage stable lyophilized collagen product comprising acid soluble purified native collagen in combination with platelet growth factors and a pharmaceutical composition for enhancing wound healing comprising an aqueous solution of water soluble acid-soluble purified native collagen and platelet derived growth factors. Said product may also comprise a perfluorocarbon, compound and a non-toxic pharmacologically acceptable emulsifying agent therefor.

Therefore, a need of finding a solution to the extremely widespread and often disabling, for long periods of time, problem of skin lesions at different degrees of severity and dermal extension is strongly present and deeply felt.

It was surprisingly found out that a medicament for topical use can be obtained by a suitable combination of disinfectant agents and tissue regenerating agents, which can heal a skin lesion within conveniently short times.

Therefore, it is an object of the present invention to provide a medicament for topical use, that is effective in rapid and resolutive treatment of skin lesions, while exhibiting considerably increased performances with respect to the treatment techniques currently in use.

A second object of the invention is to provide a medication for topical use which uses such a medicament.

A further object of the invention is to provide a kit for the preparation of such a medicament at the moment of the use.

Yet another object of the invention is to provide a process for the preparation of such a medicament.

The above objects have been achieved by a medicament for topical use obtained by the combination of suitable ingredients, as indicated in claim 1.

The medicament for topical administration according to the present invention comprises tissue regenerating agents, oxygen and ozone, carried by a fluorinated hydrocarbon, and at least one biocompatible organic compound, collagen.

In the present invention, the term "collagen" refers to heterologous and/or autologous collagen of type I and/or type II. In the present invention, the amount of collagen is expressed in area units (cm²) of collagen, having a thickness of about 5 mm.

Further features and advantages of the invention will be apparent from the following detailed description, with reference to examples of carrying out the invention, given for illustrating and non-limiting purpose, and to the annexed drawing, in which:

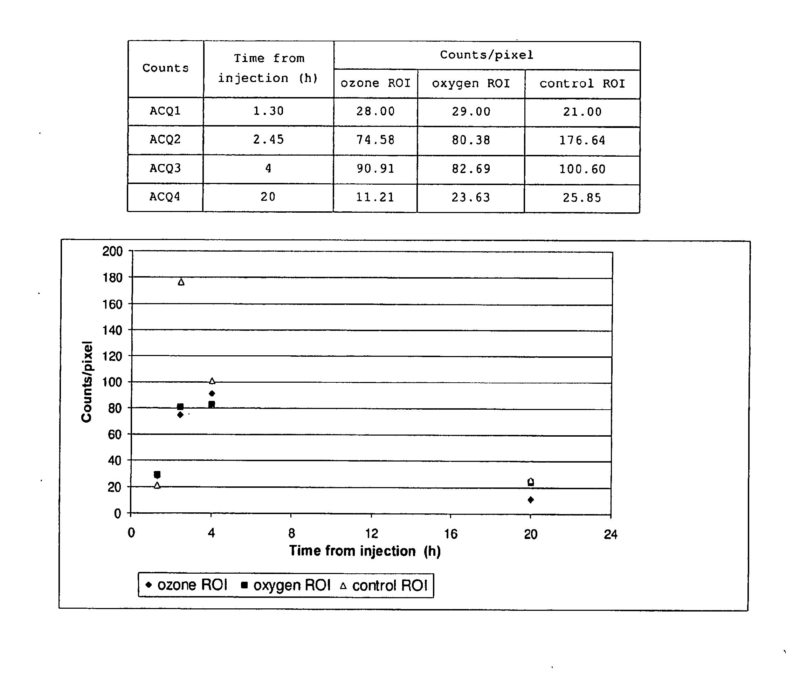

Figure 1 shows comparative evolution of the leukocyte response over time, concerning skin lesions present on the same patient and treated by using three different medications: control ROI (Δ), control oxygen ROI (▩), oxygen and ozone ROI (◆).

Therefore, the invention relates to a medicament for topical administration comprising collagen and a tissue regenerating agent, oxygen and ozone, suitably provided by a fluorinated hydrocarbon which is capable of carrying and releasing it in a favourable amount.

Said fluorinated hydrocarbons have been studied as potential blood substitutes for their capacity of carrying and releasing oxygen to tissues. For example, such fluorinated hydrocarbons are known to be used as autologous blood substitutes (European patent N. 0 627 913, published on September 2, 1993) or as components of aqueous emulsions for medical applications, such as transfusions, treatment of myocardial ischemia or conservation of organs intended for transplant (Riess, J. G. "Blood substitutes and other potential biomedical applications of fluorinated colloids", J. Of Fluorine Chemistry, 114 (2002) 119-126).

Preferably according to the invention, the fluorinated hydrocarbon carries at least 50% by weight of oxygen, based on the weight of the hydrocarbon. More preferably, the fluorinated hydrocarbon is a fluoroalkyl bromide. Most preferably, the fluorinated hydrocarbon is a perfluoro-octyl bromide of formula CF₃(CF₂)₇Br.

Preferably, an amount in cc of fluorinated hydrocarbon is used, carrying a percent amount by weight based on the weight of the hydrocarbon.

The amount of collagen is proportional to the amount of hydrocarbon and to the lesion extension in area units. Preferably the ratio of the amount of collagen to the amount of hydrocarbon is from about 1:3 to about 1:2 and the amount of collagen is advantageously sufficient to cover the almost whole extension of the lesion. More preferably, the ratio of the amount of collagen to the amount of hydrocarbon is about 1:2 and the ratio of the surface lesion extension to the amount of collagen is about 1:3.

The collagen according to the invention can be bovine collagen, swine collagen, human heterologous and/or autologous collagen, and preferably it is equine collagen of type I, more preferably equine collagen of type I in form of 5 × 5 cm² tablets having a thickness of 5 mm, even more preferably of less than 5 mm.

The medicament of the invention further comprises ozone. Such an embodiment exhibits advantageous effects in the case of skin lesions showing inflammations and/or infections or necrotic and/or purulent areas, by virtue of the bactericidal and disinfecting properties of ozone.

In a preferred embodiment of the medicament, the fluorinated hydrocarbon further carries and releases CO₂. Such an aspect results extremely advantageous, because CO₂, together with oxygen, contributes to a proper skin transpiration process at the moment of the application of the medicament. Preferably, the fluorinated hydrocarbon carries at least 200% by weight of CO₂, based on the weight of the hydrocarbon.

In a further preferred embodiment of the medicament, this one further comprises gelatin and its derivatives. Gelatin is used, together with collagen, for its hemostatic properties.

In a second aspect, the invention relates to a medication for topical administration, comprising the above medicament and a biocompatible support, according to claim 9.

Preferably, the biocompatible support is protective towards damaged skin. For instance, it can be a polyurethane film or a sterile fabric, which supports the skin transpiration. Further suitable biocompatible supports can be also provided, such as mixed collagen-gelatin supports, hydrocolloid plates, modified polymers of starch-glycerol, facultatively in a gelled form. Even more preferably, the biocompatible support is a collagen-based support.

In an other aspect, the invention relates to a kit for preparing said medicament at the moment of the use, according to claim 12.

Said kit comprises collagen, fluorinated hydrocarbon carrying and releasing both oxygen and ozone, and a means for taking and releasing said two ingredients to contact each other, such as a syringe. The collagen is preferably sterilely packed and sealed with inert plastic material, whereas the fluorinated hydrocarbon carrying and releasing both oxygen and ozone is provided in a glass vial.

Alternatively, the kit comprises the fluorinated hydrocarbon carrying and releasing oxygen, in a glass vial whose cap has a rubbery diaphragm. In this way, it is possible to take ozone from the ozonizer and injected it into the vial containing the fluorinated hydrocarbon already added with oxygen.

In a further embodiment, said kit comprises collagen, gelatin and derivatives thereof, fluorinated hydrocarbon carrying and releasing oxygen and, optionally, ozone, and at least one means for taking and releasing said ingredients to contact each other, such as a syringe. The gelatin and derivatives thereof can be premixed with collagen.

In a further aspect, the invention concerns a process for preparing said medicament, according to claim 19.

The process for preparing the medication according to the invention comprises the following steps of:
a) applying collagen on a biocompatible support;
b) adding fluorinated hydrocarbon carrying and releasing oxygen on the collagen of step a).

Such a process further comprises a step of adding ozone into the fluorinated hydrocarbon carrying and releasing oxygen.

Preferably, the step of adding fluorinated hydrocarbon takes place in two stages: a first amount is applied on the biocompatible support before collagen, whereas a second amount is subsequently added on said collagen.

Alternatively, according to claim 20 the fluorinated hydrocarbon results to be already carrying both oxygen and ozone.

In yet another aspect, the invention relates to the use of the medicament of the invention for making a medicament for skin lesion care, according to claim 22. Such lesions can be of various degrees of severity, such as bedsores, skin ulcers or the like, even made worse by infections.

The invention will be now described in detail with reference to specific examples of carrying out the medicament, the ready-to-use kit and preparation process thereof.

### Comparative Example 1. Preparation of an oxygen-comprising medication

Sterilized equine collagen of type I (5 × 5 cm²) of 5 mm thickness, was placed on a 14 × 10 cm biocompatible polyurethane support. 2 cc of perfluoro-octyl bromide CF₃(CF₂)₇Br (provided in a glass container sealed with O₃ resistant rubber and aluminum ring) with 8 cc of 100% isobaric oxygen were added.

### Example 2. Preparation of the oxygen and ozone-comprising medication according to the invention

The above example was repeated, wherein perfluoro-octyl bromide carried 50% by weight of oxygen and 50% by weight of ozone, based on the weight of the perfluoro-octyl bromide.

### Comparative Example 3. Oxygen-comprising kit for skin lesion care

The kit contained: n.3 sheets of equine collagen of type I (5 × 5 cm²), n.1 10 cc vial containing 2 cc of perfluoro-octyl bromide carrying 50% by weight of oxygen, and n.1 10 cc syringe with 21 GA 11/2 needle.

### Example 4. Oxygen and ozone-comprising kit for skin lesion care

The kit contained: n.3 sheets of equine collagen of type I (5 × 2 cm²), n.1 10 cc vial containing 2 cc of perfluoro-octyl bromide carrying 50% by weight of oxygen and 60 gamma of ozone in oxygen, and n.1 10 cc syringe with 21 GA 11/2 needle. It should be noted that ozone had been previously added to the vial of perfluoro-octyl bromide by drawing 8 cc of air and subsequently introducing 8 cc of ozone therein.

### Example 5. In vitro validation procedure

Pure cultures of salmonella bacteria (1,750 colonies) from human pathogenic expectoration and of Candida albicans fungi (60,000 colonies) from a mycological collection were used. These cultures have been thermostated at 37°C for 18 hours on bovine brain infusion broth substrate in order to ensure the maximum viability. 1 cc samples were taken from the above cultures and placed on the following dishes:
- control dish;
- dish holding 1 cm² of the control medication obtained according to Example 1;
- dish holding 1 cm² of the medication obtained according to Example 2.

The above dishes were incubated for 24, 48 and 72 hours, while monitoring the growth of colonies by a colony counter. The dishes according to the invention exhibited equivalent growth in U.F.C. of both colonies, as in the control dish, thus showing that the medications according to the invention do not promote the bacterial and fungus growth.

### Example 6. In vivo validation procedure

The following three medications were evaluated:
- control ROI: patch medication with gauze;
- control oxygen ROI: medication according to the invention, comprising collagen and perfluoro-octyl bromide in a ratio of 1 cm² of collagen to 0.5 cc perfluoro-octyl bromide carrying about 50% by weight of oxygen based on the weight of perfluoro-octyl bromide;
- oxygen and ozone ROI: medication according to the invention, comprising collagen and perfluoro-octyl bromide in a ratio of 1 cm² of collagen to 0.38 g perfluoro-octyl bromide carrying about 50% by weight of oxygen, based on the weight of perfluoro-octyl bromide, and about 49% by weight of ozone in oxygen (80 gamma), based on the weight of the perfluoro-octyl bromide.

The three above medications have been applied on three skin lesions, caused by a diathermy loop at 600°C, with a loop depth of 3 mm, on a side face of the thighs of an about 50 year-old male individual.

Said lesions were cleaned and disinfected before applying the medications, by using a common disinfectant, such as a quaternary ammonium salt solution.

The evolution of the lesions were monitored by scintigraphy with labelled leukocytes to evaluate the leukocyte inflammatory stimulus.

In order to carry out such a labelling, a 20 cc blood sample was previously taken from the individual, followed by a leukocyte separation and technetium (⁹⁹Tc) labelling. The so-labeled leukocytes were subsequently reinfused in the individual in a total dose of 766 Mega bequerel immediately before the application of the three medications.

Detections ACQ1, ACQ2, ACQ3, ACQ4 were made by means of a gamma camera at 1 hour 30 minutes (ACQ1), 2 hours 45 minutes (ACQ2), 4 hours (ACQ3) and 20 hours (ACQ4) from the reinfusion of labeled leukocytes.

With reference to Figure 1, in the detection ACQ1, slightly higher leukocyte response values have been observed for control medications with oxygen (control oxygen ROI (▩)) and medications with oxygen-ozone (oxygen and ozone ROI (◆)).

Advantageously, leukocyte response at detections ACQ2 and ACQ3 was remarkably lower for control oxygen ROI and ozone ROI medications with respect to the control ROI (Δ). The oxygen and ozone ROI was particularly advantageous, by showing a leukocyte response value lower than the control oxygen ROI and extremely low with respect to the control ROI. Such an extremely advantageous response of oxygen and ozone ROI was also observed at detection ACQ4.

It was also apparent that, the oxygen and ozone ROI lesion healing, which resulted from a low leukocyte response value, was advantageously faster than for the control and control oxygen ROI. Particularly after 20 hours, the oxygen and ozone ROI exhibited a leukocyte response value lower than half of the corresponding control and control oxygen ROI value.

The above examples and particularly Figure 1 clearly show the surprising results afforded by this invention, which allows to keep moist the environment on contact with the skin lesion, permits a proper transpiration, ensures thermal insulation, does not contain toxic or allergenic components, is sterile, forms an effective barrier against external microorganisms, does not adhere to the damaged skin area, provides an adequate mechanical protection, is comfortable and does not cause any additional pain, conforms to irregular surfaces, is simple and safe to use, requires conveniently long replacement intervals, allows the repairing process to be monitored without removing the medication.

Without to be bound by any particular theory, the in vivo effectiveness test showed that treatment by using the medicament promotes the generation and preservation of a favorable microclimate around the skin lesions. Concerning ozone, an action on metalloproteases by collagen is presumed, while protecting the growth factors and simultaneously inactivating the free radicals by ozone. Therefore, the use of such a medicament is suitable in the treatment of any lesion deriving from a surgical operation, in procedures to prevent any possible necrotic evolution of lesions, in diabetic ulcers, in venous ulcers, in radiotherapy ulcers and in radiodermatites.

## Claims

1. A product comprising collagen and a fluorinated hydrocarbon carrying and releasing oxygen and ozone, for use as a medicament for topical administration.

2. The product according to claim 1, wherein the fluorinated hydrocarbon is a fluorinated hydrocarbon carrying at least 50% by weight of oxygen, based on the weight of the hydrocarbon.

3. The product according to claim 1 or 2, wherein the fluorinated hydrocarbon further carries and releases CO₂.

4. The product according to claim 3, wherein the fluorinated hydrocarbon carries at least 200% by weight of CO₂, based on the weight of the hydrocarbon.

5. The product according to claims 1 to 4, wherein the fluorinated hydrocarbon is a fluoro-alkyl bromide.

6. The product according to claim 5, wherein the fluorinated hydrocarbon is perfluoro-octyl bromide.

7. The product according to any one of claims 1 to 6, wherein the collagen is equine collagen.

8. The product according to any one of claims 1 to 7, further comprising gelatin and the derivatives thereof.

9. A medication comprising the product according to any one of claims 1 to 8 and a biocompatible support.

10. The medication according to claim 9, wherein the support is selected from a polyurethane film and a sterile fabric.

11. The medication according to claim 9, wherein the biocompatible support is collagen-based.

12. A kit for skin lesion care comprising collagen, a fluorinated hydrocarbon carrying and releasing oxygen and ozone, and a means for taking and releasing said fluorinated hydrocarbon, wherein the collagen and the fluorinated hydrocarbon are in separate compartments, which may be equal or different and are intended for simultaneous, separate or sequential topical administration in the treatment of skin lesions.

13. The kit according to claim 12, wherein the means for taking and releasing said fluorinated hydrocarbon is a syringe.

14. The kit according to claim 12 or 13, further comprising gelatin and the derivatives thereof.

15. The kit according to any one of claims 12 to 14, wherein the fluorinated compound is a fluorinated hydrocarbon carrying at least 50% by weight of oxygen, based on the weight of the hydrocarbon.

16. The kit according to any one of claims 12 to 15, wherein the fluorinated hydrocarbon is a fluoro-alkyl bromide.

17. The kit according to any one of claims 12 to 16, wherein the fluorinated hydrocarbon is a perfluoro-octyl bromide.

18. The kit according to any one of claims 12 to 17, wherein the collagen is equine collagen.

19. A process for preparing a medication according to any one of claims 9 to 11, comprising the steps of:
a) applying collagen on a biocompatible support;
b) adding a fluorinated hydrocarbon carrying and releasing oxygen on the collagen of step a);
c) adding ozone to the fluorinated hydrocarbon carrying and releasing oxygen.

20. A process for preparing a medication according to any one of claims 9 to 11, comprising the steps of:
a) applying collagen on a biocompatible support;
b) adding a fluorinated hydrocarbon carrying and releasing oxygen and ozone on the collagen of step a).

21. The process according to claim 19 or 20, wherein the step of adding the fluorinated hydrocarbon takes place in two stages: a first amount is applied on the biocompatible support before collagen, and a second amount is subsequently added on said collagen.

22. Use of the product according to any one of claims 1 to 8, for the manufacture of a medicament for topical administration in the treatment of skin lesions.

23. The use according to claim 22, wherein the medicament is in the treatment of bedsores.

24. The use according to claim 22, wherein the medicament is in the treatment of skin ulcers.

25. The use according to claim 22, wherein the medicament is in the treatment of diabetic ulcers, venous ulcers, radiotherapy ulcers and radiodermites.

26. The use according to claim 22, wherein the medicament is in the treatment of skin lesions showing inflammations and/or infections.

27. The use according to claim 22, wherein the medicament is in the treatment of skin lesions showing necrotic and/or purulent areas.

## Patentansprüche

1. Produkt, umfassend Kollagen und einen Fluorkohlenwasserstoff, der Sauerstoff und Ozon schleppt und abgibt, zur Verwendung als Medikament zur topischen Verabreichung.

2. Produkt nach Anspruch 1, wobei der Fluorkohlenwasserstoff ein solcher Fluorkohlenwasserstoff ist, der mindestens 50 Gew.% Sauerstoff, bezogen auf das Gewicht des Kohlenwasserstoffs, schleppt.

3. Produkt nach Anspruch 1 oder 2, wobei der Fluorkohlenwasserstoff ferner CO₂ schleppt und freisetzt.

4. Produkt nach Anspruch 3, wobei der Fluorkohlenwasserstoff mindestens 200 Gew.% CO₂, bezogen auf das Gewicht des Kohlenwasserstoffs, schleppt.

5. Produkt nach den Ansprüchen 1 bis 4, wobei der Fluorkohlenwasserstoff ein Fluoralkylbromid ist.

6. Produkt nach Anspruch 5, wobei der Fluorkohlenwasserstoff Perfluoroctylbromid ist.

7. Produkt nach einem der Ansprüche 1 bis 6, wobei das Kollagen equines Kollagen ist.

8. Produkt nach einem der Ansprüche 1 bis 7, des Weiteren Gelatine und die Derivate davon umfassend.

9. Arzneimittel, umfassend das Produkt nach einem der Ansprüche 1 bis 8 und einen biokompatiblen Träger.

10. Arzneimittel nach Anspruch 9, wobei der Träger aus einem Polyurethanfilm und einem sterilen Gewebe ausgewählt ist.

11. Arzneimittel nach Anspruch 9, wobei der biokompatible Träger auf Kollagen basiert.

12. Kit zur Behandlung von Hautläsionen, umfassend Kollagen, einen Fluorkohlenwasserstoff, der Sauerstoff und Ozon schleppt und freisetzt, und eine Einrichtung zum Aufnehmen und Abgeben des Fluorkohlenwasserstoffs, wobei sich das Kollagen und der Fluorkohlenwasserstoff in separaten Kammern befinden, die gleich oder unterschiedlich sein können und für eine gleichzeitige, separate oder sequenzielle topische Verabreichung bei der Behandlung von Hautläsionen vorgesehen sind.

13. Kit nach Anspruch 12, wobei die Einrichtung zum Aufnehmen und Abgeben des Fluorkohlenwasserstoffs eine Spritze ist.

14. Kit nach Anspruch 12 oder 13, ferner Gelatine und die Derivate davon umfassend.

15. Kit nach einem der Ansprüche 12 bis 14, wobei die Fluorverbindung ein Fluorkohlenwasserstoff ist, der mindestens 50 Gew.% Sauerstoff, bezogen auf das Gewicht des Kohlenwasserstoffs, schleppt.

16. Kit nach einem der Ansprüche 12 bis 15, wobei der Fluorkohlenwasserstoff ein Fluoralkylbromid ist.

17. Kit nach einem der Ansprüche 12 bis 16, wobei der Fluorkohlenwasserstoff ein Perfluoroctylbromid ist.

18. Kit nach einem der Ansprüche 12 bis 17, wobei das Kollagen equines Kollagen ist.

19. Verfahren zum Herstellen eines Arzneimittels nach einem der Ansprüche 9 bis 11, umfassend die folgenden Schritte:
a) Aufbringen von Kollagen auf einen biokompatiblen Träger;
b) Hinzufügen eines Fluorkohlenwasserstoffs, der Sauerstoff schleppt und abgibt, zu dem Kollagen vom Schritt a);
c) Hinzugeben von Ozon zu dem Fluorkohlenwasserstoff, der Sauerstoff schleppt und abgibt.

20. Verfahren zum Herstellen eines Arzneimittels nach einem der Ansprüche 9 bis 11, umfassend die folgenden Schritte:
a) Aufbringen von Kollagen auf einen biokompatiblen Träger;
b) Hinzufügen eines Fluorkohlenwasserstoffs, der Sauerstoff und Ozon schleppt und abgibt, zu dem Kollagen vom Schritt a).

21. Verfahren nach Anspruch 19 oder 20, wobei der Schritt des Hinzufügens des Fluorkohlenwasserstoffs in zwei Teilschritten erfolgt: Eine erste Menge wird vor dem Kollagen auf den biokompatiblen Träger aufgebracht und eine zweite Menge wird nach dem Kollagen hinzugefügt.

22. Verwendung des Produkts nach einem der Ansprüche 1 bis 8 zur Herstellung eines Medikaments zur topischen Verabreichung bei der Behandlung von Hautläsionen.

23. Verwendung nach Anspruch 22, wobei das Medikament bei der Behandlung von wundgelegenen Stellen eingesetzt wird.

24. Verwendung nach Anspruch 22, wobei das Medikament bei der Behandlung von Geschwüren der Haut eingesetzt wird.

25. Verwendung nach Anspruch 22, wobei das Medikament bei der Behandlung von diabetischen Geschwüren, Venengeschwüren, durch Strahlenbehandlung hervorgerufenen Geschwüren und Strahlendermatitis eingesetzt wird.

26. Verwendung nach Anspruch 22, wobei das Medikament bei der Behandlung von Hautläsionen, die Entzündungen und/oder Infektionen aufweisen, eingesetzt wird.

27. Verwendung nach Anspruch 22, wobei das Medikament bei der Behandlung von Hautläsionen, die nekrotische und/oder eitrige Bereiche aufweisen, eingesetzt wird.

## Revendications

1. Un produit comprenant du collagène et un hydrocarbure fluoré transportant et libérant de l'oxygène et de l'ozone, à utiliser comme un médicament pour administration topique.

2. Le produit selon la revendication 1, **caractérisé en ce que** l'hydrocarbure fluoré est un hydrocarbure fluoré transportant au moins 50 % par poids d'oxygène, selon le poids de l'hydrocarbure.

3. Le produit selon la revendication 1 ou 2, **caractérisé en ce que** l'hydrocarbure fluoré transporte et libère en outre du CO₂.

4. Le produit selon la revendication 3, **caractérisé en ce que** l'hydrocarbure fluoré transporté au moins 200 % par poids de CO₂, selon le poids de l'hydrocarbure.

5. Le produit selon les revendications de 1 à 4, **caractérisé en ce que** l'hydrocarbure fluoré est un bromure fluoroalkyle.

6. Le produit selon la revendication 5, **caractérisé en ce que** l'hydrocarbure fluoré est du bromure de perfluoro-octyle.

7. Le produit selon l'une quelconque des revendications de 1 à 6, **caractérisé en ce que** le collagène est du collagène équin.

8. Le produit selon l'une quelconque des revendications de 1 à 7, comprenant en outre de la gélatine et des dérivés de celle-ci.

9. Une médication comprenant le produit selon l'une quelconque des revendications de 1 à 8 et un support biocompatible.

10. La méditation selon la revendication 9, **caractérisée en ce que** le support est sélectionné parmi un film polyuréthane et un tissu stérile.

11. La médication selon la revendication 9, **caractérisée en ce que** le support biocompatible est à base de collagène.

12. Un kit de soins pour lésions cutanées comprenant du collagène, un hydrocarbure fluoré transportant et libérant de l'oxygène et de l'ozone, et un moyen pour prélever et libérer ledit hydrocarbure fluoré, **caractérisé en ce que** le collagène et l'hydrocarbure fluoré sont dans des compartiments séparés, qui peuvent être identiques ou différents et sont prévus pour une administration topique simultanée, séparée ou séquentielle dans le traitement de lésions cutanées.

13. Le kit selon la revendication 12, **caractérisé en ce que** le moyen servant à prélever et à libérer ledit hydrocarbure fluoré est une seringue.

14. Le kit selon la revendication 12 ou 13, comprenant en outre de la gélatine et des dérivés de celle-ci.

15. Le kit selon l'une quelconque des revendications de 12 à 14, **caractérisé en ce que** le composé fluoré est un hydrocarbure fluoré transportant au moins 50 % par poids d'oxygène, selon le poids de l'hydrocarbure.

16. Le kit selon l'une quelconque des revendications de 12 à 15, **caractérisé en ce que** l'hydrocarbure fluoré est un bromure fluoroalkyle.

17. Le kit selon l'une quelconque des revendications de 12 à 16, **caractérisé en ce que** l'hydrocarbure fluoré est un bromure de perfluoro-octyle.

18. Le kit selon l'une quelconque des revendications de 12 à 17, **caractérisé en ce que** le collagène est du collagène équin.

19. Un procédé de préparation d'une médication selon l'une quelconque des revendications de 9 à 11, comprenant les phases consistant à :
a) appliquer du collagène sur un support biocompatible ;
b) ajouter un hydrocarbure fluoré transportant et libérant de l'oxygène sur le collagène de la phase a) ;
c) ajouter de l'ozone à l'hydrocarbure fluoré transportant et libérant de l'oxygène.

20. Un procédé de préparation d'une médication selon l'une quelconque des revendications de 9 à 11, comprenant les phases consistant à :
a) appliquer du collagène sur un support biocompatible ;
b) ajouter un hydrocarbure fluoré transportant et libérant de l'oxygène et de l'ozone sur le collagène de la phase a).

21. Le procédé selon la revendication 19 ou 20, **caractérisé en ce que** la phase d'addition de l'hydrocarbure fluoré a lieu en deux temps : une première quantité est appliquée sur le support biocompatible avant le collagène, et une deuxième quantité est ensuite ajoutée sur ledit collagène.

22. Utilisation du produit selon l'une quelconque des revendications de 1 à 8, pour la fabrication d'un médicament à administration topique dans le traitement de lésions cutanées.

23. L'utilisation selon la revendication 22, **caractérisée en ce que** le médicament est utilisé dans le traitement des escarres.

24. L'utilisation selon la revendication 22, **caractérisée en ce que** le médicament est utilisé dans le traitement des ulcères cutanés.

25. L'utilisation selon la revendication 22, **caractérisée en ce que** le médicament est utilisé dans le traitement des ulcères diabétiques, ulcères veineux, ulcères après radiothérapie et radiodermites.

26. L'utilisation selon la revendication 22, **caractérisée en ce que** le médicament est utilisé dans le traitement de lésions cutanées présentant des inflammations et/ou infections.

27. l'utilisation selon la revendication 22, **caractérisé en ce que** le médicament est utilisé dans le traitement de lésions cutanées présentant des zones nécrotiques et/ou purulentes.
